**Europäisches Patentamt**

**European Patent Office**

**Office européen des brevets**

(19)

(11) Veröffentlichungsnummer : **0 270 900 B1**

(12) # EUROPÄISCHE PATENTSCHRIFT

(45) Veröffentlichungstag der Patentschrift :
**05.06.91 Patentblatt 91/23**

(51) Int. Cl.⁵ : **B05D 3/00**, B05D 7/14,
A61C 13/00

(21) Anmeldenummer : **87117253.2**

(22) Anmeldetag : **24.11.87**

(54) Verfahren zur Herstellung von Zahnprothesen.

(30) Priorität : **11.12.86 DE 3642290**

(43) Veröffentlichungstag der Anmeldung :
**15.06.88 Patentblatt 88/24**

(45) Bekanntmachung des Hinweises auf die
Patenterteilung :
**05.06.91 Patentblatt 91/23**

(84) Benannte Vertragsstaaten :
**AT CH FR GB LI SE**

(56) Entgegenhaltungen :
**DE-A- 2 732 753**
**GB-A- 1 536 238**
**GB-A- 2 082 478**
**US-A- 3 657 003**
**US-A- 4 243 721**

(73) Patentinhaber : **Ivoclar AG**
**FL-9494 Schaan (LI)**

(72) Erfinder : **Rheinberger, Volker, Dr.**
**Schlossstrasse 130**
**FL-9490 Vaduz (LI)**
Erfinder : **Wollwage, Peter**
**Auf Berg 113**
**FL-9493 Mauren (LI)**
Erfinder : **Zanghellini, Gerhard**
**Schaanerstrasse 80**
**FL-9490 Vaduz (LI)**

(74) Vertreter . **UEXKÜLL & STOLBERG**
**Patentanwälte**
**Beselerstrasse 4**
**W-2000 Hamburg 52 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Zahnprothesen unter Verbesserung der Haftung des Kunststoffs auf dem Metallsubstrat gemäß dem Oberbegriff des Anspruchs 1.

Die GB-A-20 82 478 beschreibt ein Verfahren zum Beschichten von Metallsubstraten mit Kunststoffen, bei welchem die Metalloberfläche zunächst mit einem die Benetzung fördernden Hydrosol und anschließend mit einem Kupplungsmittel behandelt wird. Zur Erzielung einer möglichst guten Haftung soll nur eine monomolekulare Schicht des Hydrosols aufgebracht werden (Seite 3, Zeilen 4 bis 8). Als Beispiele für geeignete Hydrosole werden nur diejenigen des Eisens und Zinns genannt. Die Aufbringung erfolgt in der Weise, daß das Metallsubstrat mit dem Hydrosol kontaktiert und anschließend gespült wird. Gegebenenfalls kann vor Behandlung mit dem Kupplungsmittel bei einer Temperatur von 130°C getrocknet werden. Ein Einbrennen des Hydrosols findet nicht statt.

Ein Verfahren zum Aufbringen einer Kronenverblendung auf einen metallischen Zahnersatz ist aus der DE-A-32 11 123 bekannt. Der metallische Grundkörper wird ein- oder mehrmals in eine Silanlösung eingetaucht und danach getrocknet. Auf die erhaltene Silanschicht wird gleichmäßig dünn ein Prothesenkunststoff aufgetragen und anschließend die nach bekannten Verfahren hergestellte Verblendschale aufgedrückt, d.h. der Prothesenkunststoff wird auspolymerisiert. Vor der Silanisierung des metallischen Grundkörpers, die in einem Ultraschallbad ausgeführt wird, erfolgt eine Aufrauhung des metallischen Grundkörpers durch Sandstrahlung. Nachteil dieser Arbeitsweise ist es, daß lediglich Si-haltige Nichtedelmetallegierungen und nur keramische Verblendschalen verwendet werden können.

Weiterhin beschreibt die US-A-4 364 731 eine Haftvermittlerschicht aus anorganischen Oxiden, darunter auch Siliciumdioxid. Diese Oxidschicht wird silanisiert und es wird das Verblendmaterial nach bekannten Verfahren aufgebracht. Die anorganische Oxidschicht wird mittels einer sogenannten Sputter-Vorrichtung auf die Metalloberfläche aufgetragen. Dieses Verfahren verlangt eine äußerst präzise Vorgehensweise zur Herstellung einer unbeschädigten Mantelschicht aus anorganischen Oxiden. Weitere in der gleichen Patentschrift vorgeschlagene Verfahren sind die Beschichtung aus der Dampfphase unter chemischer Zersetzung (CVD-Verfahren) und die Plattierung mit Hilfe reaktiver Ionen.

In der DE-C-34 03 894 findet sich eine Abwandlung der Arbeitsweise der US-Patentschrift, die darin besteht, daß die Siliciumdioxidschicht mit Hilfe eines Flammhydrolysebrenners unter Verwendung einer gasförmigen oxidierbaren Siliciumverbindung aufgebracht wird.

Die oben erwähnten Vorschläge des Standes der Technik sind entweder nur für bestimmte in der Zahnprothetik verwendete Werkstoffkombinationen zu verwenden oder sie benötigen einen hohen, in zahntechnischen Labors normalerweise nicht akzeptablen apparativen Aufwand und zudem eine exzellente Beherrschung des Verfahrens zur Erlangung einer optimalen Haftvermittlerschicht. So müssen bestimmte Bedingungen wie z.B. der Abstand des Flammhydrolysebrenners oder der Sputter-Vorrichtung vom Werkstück, nämlich dem metallenen Zahnersatz, genau eingehalten werden.

Der Erfindung liegt demgegenüber die Aufgabe zugrunde, ein Verfahren zur Erzeugung einer Haftvermittlerschicht für Kunststoff/Metall bei der Herstellung von Zahnprothesen bereitzustellen, welches die oben genannten Nachteile nicht aufweist, sowie leicht und ohne großen apparativen Aufwand durchführbar ist. Weiterhin ist es Aufgabe der Erfindung, dem mit der Zeit zwischen Metall und Kunststoff durch die verschiedenen Ausdehnungskoeffizienten entstehenden Randspalt, der zu unschönen Verfärbungen führt, entgegenzuwirken und eine unlösbare Verbindung zwischen Metall und Kunststoff herzustellen

Gegenstand der Erfindung ist ein Verfahren zur Herstellung von Zahnprothesen, bei welchem man unter Verbesserung der Haftung des Kunststoffs auf dem Metallsubstrat auf die Metalloberfläche eine Siliciumdioxidschicht aufbringt und diese anschließend silanisiert, welches dadurch gekennzeichnet ist, daß man die Siliciumdioxidschicht durch Auftragen eines Kieselsols oder einer Dispersion einer feinstteiligen Kieselsäure auf die Metalloberfläche und Einbrennen bei einer Temperatur von 300 bis 800°C aufbringt.

Das erfindungsgemäße Verfahren weist mehrere wesentliche Vorteile auf :

– Auf die Oberfläche von Metallstücken für Kronen oder Brücken wird nach Sandstrahlung ein Kieselsäuresol in Wasser in dünner Schicht lediglich aufgepinselt, getrocknet und bei Temperaturen von 300 bis 800°C eingebrannt.

– Das wässrige Kieselsol ist eine stabile nicht sedimentierende Suspension, die käuflich im Handel erhalten werden kann

– Eine Kieselsäuredispersion kann auch aus feinstteiliger Kieselsäure, z.B. pyrogener Kieselsäure, und $H_2O$ hergestellt werden.

– Das Verfahren benötigt weder eine Sputter-Vorrichtung noch einen Flammhydrolysebrenner zur Aufbringung der Siliciumdioxidteilchen, sondern nur einen in jedem Dental-Labor üblichen Keramikofen zum Einbrennen.

2

– Alle anderen Verfahren, die keramische Materialien verwenden, benötigen

a) Legierungen, die für Keramik geeignet sind und

b) Temperaturen zum Aufbrennen, die über der für Kronen-und Brücken-Legierungen zulässigen Temperatur liegen.

Die erfindungsgemäß verwendeten Kieselsole sind wässrige, kolloidale Dispersionen von amorphem Siliciumdioxid. Das Siliciumdioxid liegt in Form von untereinander unvernetzten, kugelförmigen Einzelpartikeln vor, die an der Oberfläche Hydroxylgruppen aufweisen. Die Kieselsole sind homogene, sich nicht entmischende, niedrig viskose Flüssigkeiten, die ohne Gesundheitsgefährdung gehandhabt werden können.

Die handelsüblichen Kieselsole enthalten Kieselsäuren mit einer durchschnittlichen Primärteilchengröße zwischen 5 und 150, insbesondere 5 und 50 nm. Die spezifische Oberfläche (BET) der Kieselsäuren liegt zwischen etwa 50 und 700 m²/g. Die Kieselsol-Typen mit einer Oberfläche von 200 bis 300 m²/g sind im allgemeinen bevorzugt, da gefunden wurde, daß mit ihrer Hilfe eine optimale Haftung zwischen Metall und Kunststoff erreicht werden kann.

Anstelle von Kieselsolen lassen sich auch Dispersionen von feinstteiliger Kieselsäure in Wasser und/oder Alkohol als Dispergiermittel verwenden. Besonders geeignet sind pyrogene Kieselsäuren mit einer durchschnittlichen Primärteilchengröße von 5 bis 50, insbesondere 5 bis 10 nm und einer spezifischen Oberfläche (BET) von 50 bis 400 m²/g. Solche Dispersionen können auch unmittelbar vor Gebrauch in üblicher Weise mit speziellen Rührern, die hohe Scherkräfte erzeugen (z.B. Polytron) hergestellt werden. Dazu ist es vorteilhaft, Stabilisatoren wie anorganische Fluorverbindungen, vorzugsweise $K_2ZrF_6$ zuzusetzen, um ein Absetzen und Eindicken zu verhindern.

Nach dem Aufbringen des Sols bzw. der Dispersion auf die Oberfläche der Metallwerkstücke werden das Wasser und/oder der Alkohol durch Trocknen entfernt, so daß eine Kieselsäureschicht zurückbleibt. Durch Einbrennen bei Temperaturen von 300 bis 800°C wird die Schicht auf der Metalloberfläche fest verankert. Die Einbrennzeit beträgt im allgemeinen etwa 2 bis 20 min., vorzugsweise 3 bis 10 min. Dabei ist die Einbrenndauer normalerweise umso länger je niedriger die Einbrenntemperatur liegt. Überraschenderweise findet bei diesen niedrigen Einbrenntemperaturen bereits eine Art Sintern statt, obwohl der Schmelzpunkt von $SiO_2$ oberhalb 1700°C liegt. Entgegen den bisherigen Annahmen ist es nicht erforderlich, die Schicht mittels Sputtern oder Flammhydrolyse aufzubringen, um einen ausreichenden Verbund mit dem metallischen Substrat zu erzielen. Im Gegenteil zeigt sich, daß die beiniedrigeren Temperaturen erzeugten erfindungsgemäßen Oxidschichten eine höhere Elastizität aufweisen und daher thermisch bedingten Dimensionsveränderungen des Metalls besser zu folgen vermögen, ohne daß es zu einer Ablösung und Spaltbildung kommt.

Die Silanisierung der eingebrannten Oxidschicht erfolgt in an sich bekannter Weise. Bevorzugte Silane sind beispielsweise Vinyltrimethoxysilan, N-β-(N-Vinylbenzylamino)ethyl-γ-aminopropyltrimethoxysilan, γ-Methacryloxypropyltrimethoxysilan, γ-Glycidyloxypropyltrimethoxysilan und andere. Diese Verbindungen werden vorzugsweise in Form alkoholischer oder essigsaurer Lösungen verwendet.

Zur näheren Erläuterung der Erfindung dienen die nachfolgenden Beispiele, in welchen anhand von Modellversuchen gezeigt wird, daß die besonders einfach durchzuführende erfindungsgemäße Arbeitsweise überraschend zu einer außergewöhnlich hohen Festigkeit des Verbundes zwischen Metallsubstrat und dem zur Verblendung dienenden Kunststoff führt.

Beispiel 1

Es werden verschiedene Kieselsole dünn auf ein Plättchen (10 × 20 × 1 mm) einer Nichtedelmetallegierung (NEM-Legierung) aufgetragen. Bei der NEM-Legierung handelt es sich um eine dentaltechnische Kobalt-Chromlegierung. Die Plättchen werden vor dem Auftragen der Kieselsole sandgestrahlt. Nach der Beschichtung werden die Plättchen getrocknet und dann 5 Minuten lang bei 400°C in einem Brennofen für Dentalkeramik (Programat) gebrannt. Es entsteht eine glasartige Schicht, die silanisiert wird. Als Silanisierungsmittel wird eine 10%-ige γ-Methacryloxipropyltrimethoxysilan-Lösung verwendet. Nach dem Silanisieren wird eine dünne opake Kunststoffschicht, die im wesentlichen aus einem fließfähigen Gemisch aus Methylmethacrylat und einem opaken Methylmethacrylatpolymer besteht, aufgetragen und 10 min lang bei 120°C trocken polymerisiert. Es entsteht eine feste Kunststoffbeschichtung, die an der Oberfläche eine dünne Schmierschicht aus nicht polymerisiertem Material aufweist.

Auf die Plättchen werden Metallröhrchen gestellt, die einen Innendurchmesser von 5 mm aufweisen. In die Metallröhrchen wird ein heißpolymerisierbares Kronen- und Brückenmaterial (ca. 40% Reaktionsprodukt aus Hydroxyethylmethacrylat und Trimethylhexamethylendiisocyanat vermischt mit einer pyrogenen Kieselsäure) eingepreßt und auspolymerisiert. Das Metallröhrchen wird abgezogen und man erhält ein Metallplättchen mit dem anpolymerisierten Kunststoffzylinder, der einen Durchmesser von 5 mm und eine Höhe von 5 mm aufweist. Nach einer Stunde Wasserlagerung bei 37°C erfolgt die Messung der Scherfestigkeit. Das Plätt-

3

chen wird dazu eingespannt und der Kunststoffzylinder wird mit einem Keil in einem Abstand von 0,5 mm vom Plättchen belastet. Der Vorschub beträgt 0,8 mm/min, die Belastung geschieht bis zum Bruch. Als Vergleich dient ein Plättchen ohne Kieselsol und Silanisierung.

Tabelle 1 zeigt die verwendeten Kieselsole, Tabelle 2 die erhaltenen Scherfestigkeitswerte. Die Kieselsole A, B, C, D und E sind käuflich erhältlich (z.B. Bayer AG), die Dispersion F wird hergestellt, indem 20 g mikrofeines Siliciumdioxid (pyrogene Kieselsäure AEROSIL 200 der Degussa AG) mit 1 g $K_2ZrF_6$ und 80 g destilliertem Wasser homogen in einem Polytronmischer vermischt werden.

## Tabelle 1

### Kieselsole

| | A | B | C | D | E |
|---|---|---|---|---|---|
| Feststoffgehalt % | 30 | 40 | 30 | 30 | 30 |
| $Na_2O$-Gehalt % | 0,15 | 0,4 | x | 0,35 | x |
| ph-Wert | 10 | 10 | 9,3 | 9,8 | 9,1 |
| Dichte g/cm³ | 1,2 | 1,29 | 1,2 | 1,21 | 1,2 |
| Viskosität mPa s | 2-3 | 7-10 | 3-6 | 3-6 | 3-4 |
| Spez. Oberfläche m²/g | 100 | 200 | 300 | 300 | 200 |
| mittl. Teilchengröße der Primärteilchen | 25-30 | 15-20 | 7-8 | 7-8 | 15-20 |
| Ionogenität | a. | a. | a. | a. | a. |

x mit $NH_3$ stabilisiert

a. = anionisch

Tabelle 2

Scherfestigkeit in N/mm$^2$

| Kieselsol | A | $11,2 \pm 2,5$ |
|-----------|---|----------------|
|  | B | $9,9 + 1,9$ |
|  | C | $13,3 \pm 1,8$ |
|  | D | $13,5 \pm 2,3$ |
|  | E | $13,0 \pm 2,4$ |
|  | F | $8,1 \pm 2,3$ |

Vergleichsbeispiel  $4,0 \pm 1,2$
(ohne Kieselsol)

Es zeigt sich, daß die Haftung des Kunststoffes auf dem Metall durch das erfindungsgemäße Verfahren signifikant verbessert wird.

### Beispiel 2

Beispiel 1 wird mit verschiedenen Edelmetallegierungen (EM-Legierungen) wiederholt. Es wurden dazu sogenannte Sparlegierungen verwendet, die als Hauptbestandteile Silber und Palladium aufweisen. Die Scherfestigkeitswerte sind in Tabelle 3 in N/mm$^2$ für 3 Kieselsole auf unterschiedlichen Legierungen angegeben.

## Tabelle 3

### Scherfestigkeit $N/mm^2$

| | Vergleich ohne Kieselsol | C | D | E |
|---|---|---|---|---|
| Leg. 1 | 3,3 ± 1,3 | 7,2 ± 1,6 | 6,8 ± 1,1 | 7,6 ± 1,7 |
| Leg. 2 | 3,3 ± 0,7 | 5,2 ± 2,7 | 7,0 ± 2,5 | 6,3 ± 1,6 |
| Leg. 3 | 2,9 ± 0,6 | 7,4 ± 2,8 | 7,8 ± 3,3 | 5,9 ± 0,9 |

Auch hier zeigt sich, daß der Verbund Kunststoff/Metall durch die erfindungsgemäße Verwendung von Kieselsolen als Haftvermittler stark verbessert wird.

## Ansprüche

1. Verfahren zur Herstellung von Zahnprothesen, bei welchem man unter Verbesserung der Haftung des Kunststoffs auf dem Metallsubstrat auf die Metalloberfläche eine Siliciumdioxidschicht aufbringt und diese anschließend silanisiert, dadurch gekennzeichnet, daß man die Siliciumdioxidschicht durch Auftragen eines Kieselsols oder einer Dispersion einer feinstteiligen Kieselsäure auf die Metalloberfläche und Einbrennen bei einer Temperatur von 300 bis 800°C aufbringt.

2. Verfahren nach Anspruch 1, dadurch gekennzeichnet, daß man eine Einbrennzeit von 2 bis 20 min. wählt.

3. Verfahren nach Anspruch 1, dadurch gekennzeichnet, daß man die Metalloberfläche durch Sandstrahlung vorbehandelt.

4. Verfahren nach einem der Ansprüche 1 bis 3, dadurch gekennzeichnet, daß man ein Kieselsol mit einer durchschnittlichen Primärteilchengröße von 5-150 nm verwendet.

5. Verfahren nach einem der Ansprüche 1 bis 5, dadurch gekennzeichnet, daß man eine Kieselsäuredispersion mit einer durchschnittlichen Primärteilchengröße von 5 bis 50 nm verwendet.

6. Verfahren nach einem der Ansprüche 1 bis 4, dadurch gekennzeichnet, daß man Wasser und/oder Alkohol als Dispergiermittel für das Kieselsol oder die Kieselsäuredispersion verwendet

7. Verfahren nach einem der Ansprüche 5 und 6, dadurch gekennzeichnet, daß man zur Stabilisierung der Kieselsäuredispersion anorganische Fluorverbindungen, insbesondere $K_2ZrF_6$ verwendet.

## Claims

1. Process for producing dental prostheses, in which a silicon dioxide layer with associated improved adhesion of the plastics material to the metal substrate is applied to the metal surface and subsequently silanised, characterised in that the silicon dioxide layer is obtained by applying a silica sol or a dispersion of very finely-divided silicic acid to the metal surface and stoving at a temperature of 300 to 800°C.

2. Process according to claim 1, characterised in that a stoving time of 2 to 20 minutes is chosen.

3. Process according to claim 1, characterised in that the metal surface is pretreated by sandblasting.

4. Process according to one of claims 1 to 3, characterised in that a silica sol with an average primary particle size of 5 to 150 nm is used.

5. Process according to one of claims 1 to 5, characterised in that a silicic acid dispersion with an average primary particle size of 5 to 50 nm is used.

6. Process according to one of claims 1 to 4, characterised in that water and/or alcohol is used as dispersant for the silica sol or silicic acid dispersion.

7. Process according to one of claims 5 and 6, characterised in that inorganic fluorine compounds, particularly $K_2ZrF_6$ are used to stabilise the silicic acid dispersion.

## Revendications

1. Procédé pour la fabrication de prothèses dentaires, améliorant l'adhérence de la matière plastique sur le substrat métallique, consistant à appliquer sur la surface métallique une couche de dioxyde de silicium qui est ensuite enduite de silane, caractérisé par le fait que la couche de dioxyde de silicium est obtenue par application d'un sol silicique ou d'une dispersion très fine d'un acide silicique sur la surface métallique, suivie d'une cuisson à une température de 300 à 800°C.

2. Procédé selon la revendication 1, caractérisé par le fait que la durée de cuisson est choisie entre 2 et 20 minutes.

3. Procédé selon la revendication 1, caractérisé par le fait que la surface métallique est soumise à un traitement préparatoire de sablage.

4. Procédé selon une des revendications 1 à 3, caractérisé par le fait que l'on utilise un sol silicique dont les particules primaires ont une taille moyenne de 5-150 nm.

5. Procédé selon une des revendications 1 à 5, caractérisé par le fait que l'on utilise une dispersion d'acide silicique dont les particules primaires ont une taille moyenne de 5-50 nm.

6. Procédé selon une des revendications 1 à 4, caractérisé par le fait que l'on utilise de l'eau et/ou de l'alcool comme agent dispersif du sol silicique ou de la dispersion d'acide silicique.

7. Procédé selon une des revendications 5 et 6, caractérisé par le fait que l'on utilise des composés inorganiques du fluor, en particulier du $K_2ZrF_6$, comme agent stabilisant de la dispersion d'acide silicique.